# EUROPÄISCHE PATENTSCHRIFT

(11) **EP 3 380 047 B1**
(45) Veröffentlichungstag und Bekanntmachung des Hinweises auf die Patenterteilung: **08.07.2020**
(21) Anmeldenummer: 16804722.3
(22) Anmeldetag: 22.11.2016
(51) Int. Cl.: A61F 2/34, A61F 2/30

(54) **GELENKPFANNENIMPLANTAT**
ACETABULAR IMPLANT
IMPLANT ACETABULAIR

(30) Priorität: 23.11.2015 EP 15195856
(43) Veröffentlichungstag der Anmeldung: 03.10.2018
(73) Patentinhaber: Jossi Holding AG, 8546 Islikon (CH)
(72) Erfinder: BÜRGI, Pascal, 8604 Volketswil (CH); GUGLER, Christian, 8500 Frauenfeld (CH); NADLER, Daniel, 8442 Hettlingen (CH)
(74) Vertreter: Hepp Wenger Ryffel AG
(86) Internationale Anmeldenummer: PCT/EP2016/078390
(87) Internationale Veröffentlichungsnummer: WO 2017/089332

(56) Entgegenhaltungen:
- EP-A2- 1 297 800
- EP-B1- 0 663 193
- WO-A2-02/064066
- FR-A1- 2 936 145
- US-A- 5 800 555

## Beschreibung

Die vorliegende Erfindung betrifft ein Gelenkpfannenimplantat, insbesondere ein Hüftgelenkpfannenimplantat, umfassend eine Aussenschale und eine Innenschale.

Beim Auftreten von Schädigungen an einer Gelenkpfanne, beispielsweise am Acetabulum, eines Patienten ist es gängige Praxis, eine künstliche Gelenkpfanne zu implantieren. Solche Gelenkpfannen sind oft zweiteilig aufgebaut, namentlich aus einer Aussenschale und einer Innenschale. Die Aussenschale dient dabei dem Einbetten der Gelenkpfanne in den Knochen. Die Innenschale, welche in die Aussenschale eingesetzt wird, bildet eine Lagerfläche für einen entsprechenden Gelenkkopf. Während Aussenschalen häufig aus einem Metall gefertigt sind, sind für Innenschalen sowohl Keramikmaterialien als auch Kunststoffe, insbesondere Polyäthylen, aber auch geeignete Metalle als Werkstoffe gebräuchlich.

Sowohl bei keramischen Innenschalen als auch bei solchen aus Kunststoff oder Metall ist es essentiell, dass eine Rotation gegenüber der Aussenschale verhindert wird. Dies liegt einerseits daran, dass es durch gegenseitige Reibung von nicht dazu vorgesehenen Teilen innerhalb der Gelenkschale rasch zu einer Beschädigung derselben kommt. Abgeriebene Partikel können zudem auf die Lagerflächen des Gelenkes gelangen und dort einen raschen Verschleiss verursachen, oder im schlimmsten Fall sogar in den Organismus des Patienten gelangen und diesen schädigen. Darüber hinaus muss insbesondere bei einseitig überhöhten Innenschalen sichergestellt werden, dass diese in einer bestimmten Winkelposition fixiert sind.

Keramische oder metallische Innenschalen sind in der Regel konisch ausgeführt, wodurch sie nach Einsetzen in eine Aussenschale eine genügend grosse Haltekraft gegen das von einem Gelenckopf übertragene Drehmoment aufbringen. Innenschalen aus einem Kunststoffmaterial müssen dagegen zusätzlich gegen ein Verdrehen gesichert sein. Viele Aussenschalen weisen daher eine Verdrehsicherung in Form von Aussparungen in ihrem Randbereich (sogenannte "Scallops") auf, in die konvexe Strukturen der Innenschale eingreifen. Jedoch ist die Fertigung solcher Gelenkpfannen aufwändig und kostenintensiv.

In jüngerer Zeit wurden daher Aussenschalen entwickelt, auf deren konkaven Innenseite stachelförmige Eindringelemente angeordnet sind, die sich beim Einsetzen einer Innenschale aus Kunststoff in diese hineinbohren und so gegen ein Verdrehen sichern. Beispielsweise offenbart die EP 0 663 193 B1 eine Revisionspfanne für ein künstliches Hüftgelenk. Die besagte Pfanne umfasst eine halbkugelförmige Stützschale und eine entsprechend dimensionierte Innenschale. Die konkave Innenseite der Stützschale ist mit Eindringelementen versehen, die beim Einsetzen der Innenschale in deren Aussenfläche eindringen, um ein Verdrehen zu verhindern. Darüber hinaus offenbart die US 5,800,555 einen Lagerkörper für eine Kugelgelenkprothese. Dieser Lagerkörper wird in eine Aussenschale eingesetzt, die auf ihrer Innenseite Vorstösse zur Rotationssicherung aufweist. Hierzu dringen die Vorstösse in die äussere Oberfläche des Lagerkörpers ein.

Derartige Verdrehsicherungen haben den Vorteil, dass zu deren Umsetzung keine konstruktiven Massnahmen an der Innenschale notwendig sind. Hingegen fallen bei der Fertigung der Aussenschale durch das Anbringen der Eindringelemente bedeutende Kosten an. Oft können die Eindringelemente nicht einstückig mit der Schale gefertigt werden, was ein Anbringen von Bohrungen und ein Setzen und Einpressen der Elemente erforderlich macht. Bestehen die Eindringelemente aus einem anderen Material als die Aussenschale, ist eine Deklaration desselben bei der Produktzulassung erforderlich, was diese zusätzlich erschwert. Um ein Einsetzen der Innenschale in die Aussenschale zu ermöglichen, müssen dornförmige Eindringelemente zudem zwangsläufig am Boden der Aussenschale angebracht sein, wodurch sie sich relativ nahe an der Längsmittelachse der Aussenschale befinden und dadurch nur ein geringes Drehmoment auf die Innenschale übertragen können.

Durch die WO 02/064066 A2 ist zudem eine implantierbare Pfanne für Hüftgelenk-Endoprothesen bekannt geworden, bei der die Lagerschale in die innere Aufnahme der Pfanne in beliebiger Drehstellung einsetzbar ist und verdrehsicher fixiert ist. Zu diesem Zweck ist am Innenumfang der Aufnahme eine zirkuläre Vielfachverzahnung vorgesehen, die zur Verdrehsicherung in die Lagerschale eingreift.

Eine ähnliche Lösung ist in der FR 2 936 145 beschrieben, wobei einzelne Zähne jeweils paarweise an einem Umfangsbereich angeordnet sind.

Ein Nachteil der bisher bekannten Rotationshemmung durch Verzahnung besteht darin, dass hohe Einpresskräfte zum Einsetzen der Innenschale erforderlich sind und dass die Gefahr besteht, dass das Material der Innenschale beschädigt wird, wobei eine nicht erwünschte Zerspanung stattfindet, bei welcher möglicherweise Partikel der Innenschale abgetragen werden. Die Zähne haben dabei eine ungünstige Konfiguration oder sie sind überdimensioniert oder zu zahlreich angeordnet, um einem vorgegebenen Solldrehmoment zwischen den beiden Schalenteilen zu widerstehen. Dabei ist ausserdem noch zu berücksichtigen, dass die zum Einsetzen der Innenschale erforderliche Einpresskraft begrenzt ist, wenn der Chirurg die Innenschale in situ einschlagen oder einpressen muss.

Es ist daher eine Aufgabe der vorliegenden Erfindung, die oben genannten Nachteile im Stand der Technik zu überwinden. Insbesondere ist es eine Aufgabe der Erfindung, ein Gelenkpfannenimplantat mit einer möglichst zuverlässigen und konstruktiv einfachen Verdrehsicherung bereitzustellen. Eine derartige Verdrehsicherung soll leicht in bestehende Implantatsysteme implementierbar sein. Zudem soll deren Implementierung kostengünstig sein und dem behandelnden Chirurgen eine leichte Handhabung des Implantates sowie eine stufenlose axiale Winkeleinstellung bezüglich der Schalenachse ermöglichen.

Weiter ist es eine Aufgabe der vorliegenden Erfindung, die Verzahnung derart auszubilden, dass das weichere Material der Innenschale nicht beschädigt wird und dass bei möglichst geringer Einpresskraft ein maximales Drehmoment übertragbar ist.

Diese Aufgaben werden durch ein Gelenkpfannenimplantat mit den Merkmalen in Anspruch 1 gelöst. Dieses Gelenkpfannenimplantat umfasst eine Aussenschale mit einer konvexen Aussenseite und einer konkaven Innenseite, welch letztere in einem kreisförmig umlaufenden Bereich eine Verzahnung aufweist. Die Flanken der Verzahnung sind parallel zur Längsmittelachse der Aussenschale ausgerichtet und das Zahnprofil weist eine Zahnhöhe auf, welche definiert ist als Differenz zwischen dem Radius des Kopfkreises und dem Radius des Fusskreises der Verzahnung. Das Gelenkpfannenimplantat umfasst ferner eine in die Aussenschale eingesetzte Innenschale, wobei die Härte der Aussenschale grösser ist als diejenige der Innenschale und wobei der mit der Verzahnung in Kontakt kommende Bereich der Innenschale relativ zum Kopfkreis der Verzahnung ein Übermass hat. Dadurch ist eine Hemmung der relativen Rotationsfähigkeit der beiden Schalen durch Verdrängen des Materials der Innenschale durch die Verzahnung erzielbar.

Es ist besonders vorteilhaft, wenn sich die Verzahnung durchgehend, vorzugsweise mit gleichmässiger Teilung, über den kreisförmig umlaufenden Bereich an der Aussenschale erstreckt. Unter gleichmässiger Teilung wird dabei ein gleicher Abstand zwischen den einzelnen Zähnen verstanden und zwar derart, dass die Verzahnung einer sich über 360° erstreckenden Innenverzahnung entspricht.

Vorteilhaft weisen die Aussenschale und die Innenschale korrespondierende axiale Rastelemente auf, an denen sie bezogen auf die gemeinsame Längsmittelachse miteinander verrastet sind. Eine derartige Verrastung ist erforderlich, weil mit einer Innenschale aus Kunststoffmaterial in axialer Richtung keine Selbsthemmung beispielsweise durch eine Konusverbindung erzielbar ist.

Besonders vorteilhaft ist der umlaufende Bereich mit der Verzahnung in einem zylindrischen Abschnitt der konkaven Innenseite der Aussenschale angeordnet, der sich bezogen auf die gemeinsame Längsmittelachse an die korrespondierenden Rastelemente anschliesst. Durch die unmittelbare Nachbarschaft der Rastelemente zu der Verzahnung können produktionstechnische Vorteile erzielt werden. Dabei spielt es keine Rolle, ob bezogen auf die gemeinsame Längsmittelachse vom Äquator der Schale zum Scheitelpunkt gesehen zuerst die Verzahnung oder die Rastelemente angeordnet sind. Es wäre grundsätzlich auch möglich, die Verzahnung und die Rastelemente bezogen auf die gemeinsame Längsmittelachse mit einem Abstand zueinander in der Aussenschale anzuordnen. Besonders vorteilhaft ist es jedoch, wenn der umlaufende Bereich mit der Verzahnung bezogen auf die Längsmittelachse näher am Äquator der Aussenschale liegt als an deren Scheitelpunkt. In diesem Äquator nahen Bereich ist der Innendurchmesser der Aussenschale am grössten, womit auch ein maximaler Durchmesser der Verzahnung erreicht werden kann. Dadurch kann sowohl die Anzahl Zähne vergrössert, als auch der Abstand der Zähne zur Drehachse vergrössert und dadurch das grösstmögliche Drehmoment übertragen werden.

Weiter ist es besonders vorteilhaft, wenn die Verzahnung bezogen auf die Längsmittelachse eine Breite zwischen 0,5 mm bis 5 mm bevorzugt zwischen 0,8 mm bis 1,6 mm aufweist. Mit der Breite der Verzahnung lässt sich in Abhängigkeit von deren Eindringtiefe in die Innenschale das übertragbare Drehmoment bestimmen. Je breiter die Verzahnung und damit der maximal mögliche Eingriff bezogen auf die Längsmittelachse ausfällt, desto höher ist das übertragbare Drehmoment an der Verzahnung.

Weiter ist es besonders vorteilhaft, wenn die Verzahnung mit einer Zahneindringtiefe in das Material der Innenschale eindringt, welche zwischen 0,1 mm bis 1 mm, bevorzugt zwischen 0,15 mm bis 0,3 mm liegt. Es hat sich überraschend gezeigt, dass die Einpresskraft im Wesentlichen durch die Zahneindringtiefe bestimmt wird und nur unbedeutend durch die vorstehend erwähnte Zahnbreite. Je höher die Eindringtiefe ist, und je mehr Zähne die Verzahnung aufweist, desto höher ist die erforderliche Einpresskraft. Die Eindringtiefe sollte jedoch so klein wie möglich ausfallen, damit keine Beschädigung oder Abspannung am Material der Innenschale stattfindet. Daraus ergibt sich die Lehre, dass die Anzahl Zähne möglichst gross und deren Eindringtiefe möglichst klein gehalten werden muss, damit die Einpresskraft tief gehalten werden kann. Das übertragbare Drehmoment kann danach mit Hilfe der Zahnbreite definiert werden.

Dabei hat es sich als besonders vorteilhaft erwiesen, wenn die Gesamtzahl Zähne der Verzahnung und die Breite der Zähne bezogen auf die Längsmittelachse derart gewählt werden, dass die Hemmung der Rotationsfähigkeit ein vorgegebenes Solldrehmoment immer übertrifft. Das minimal übertragbare Drehmoment wird durch einschlägige Medizinalnormen vorgegeben und muss in jedem Fall eingehalten werden. Bei einer vorgegebenen Eindringtiefe zwischen 0,1 mm und 1 mm und in Abhängigkeit von der Schalengrösse bzw. vom Durchmesser der Verzahnung lassen sich so die Zähnezahl und deren Breite optimieren.

Die einzelnen Zähne der Verzahnung können einen Winkel von vorzugsweise 45° bis 100° einschliessen. Aus fabrikationstechnischen Gründen wird sich an der Spitze jeweils immer eine geringfügige Abflachung oder Rundung ergeben. Ein Winkel von mehr als 100° führt zu entsprechend breiten Zähnen am Zahnfuss, womit die Gesamtzahl der Zähne reduziert wird, die am umlaufenden Bereich untergebracht werden können.

Es hat sich ferner als besonders vorteilhaft erwiesen, wenn die Seitenflanken der Zähne als ebene Flächen ausgebildet sind. Damit ist gewährleistet, dass möglichst die gesamte Zahnflanke an der Innenschale anliegt.

Weitere Vorteile können erreicht werden, wenn zwischen den Seitenflanken benachbarter Zähne und der in die Verzahnung eingesetzten Innenschale ein Hohlraum verbleibt. Dieser ermöglicht bei Innenschalen aus weichem Material eine weitere Ausdehnung, insbesondere einen Materialfluss in den Hohlraum unter Last. Auf diese Weise können schädliche Materialspannungen, welche zu einer erhöhten Einpresskraft oder einer unerwünschten Ausdehnung des Materials gegen die Längsmittelachse hin, vermieden werden.

In bestimmten Fällen kann sich die Verzahnung auch nur teilweise über den kreisförmig umlaufenden Bereich erstrecken. Eine derart teilweise unterbrochene Verzahnung hat den Vorteil, dass die Einpresskraft reduziert werden kann und zwar bei gleichbleibender Eindringtiefe.

Besonders vorteilhaft besteht die Innenschale aus einem Kunststoffmaterial, insbesondere aus einem Polyethylen mit einer Shore-Härte D zwischen 50 und 85. Ein Kunststoffmaterial mit dieser Härte weist optimale Materialeigenschaften auf, damit die Innenschale zerstörungsfrei in die Verzahnung gepresst werden kann.

Vorzugsweise ist mittels der Verzahnung ein Drehmoment von mehr als 10 Nm (Newtonmeter), vorzugsweise von mehr als 12 Nm übertragbar. Dabei handelt sich um das vorstehend erwähnte SollDrehmoment, das durch einschlägige Medizinalnormen vorgegeben ist. So beispielsweise durch das "Guidance document for testing acetabular cup prostheses" der US amerikanischen Federal Drug Administration (FDA). Die angegeben Werte enthalten einen Sicherheitsfaktor im Verhältnis zu den tatsächlich unter Last auftretenden Drehmomenten, die durch einen Patienten ausgelöst werden können.

Die Aussenschale für das Gelenkpfannenimplantat, insbesondere für ein Hüftgelenkpfannenimplantat, hat eine konvexe Aussenseite und eine konkave Innenseite zur Aufnahme einer Innenschale. Die konkave Innenseite weist in einem insbesondere kreisförmig umlaufenden Bereich zumindest teilweise eine Verzahnung auf. Durch die Verzahnung ist beim Einsetzen der Innenschale in die Aussenschale eine Hemmung der relativen Rotationsfähigkeit der beiden Schalen bezüglich der gemeinsamen Hauptachse erzielbar.

Durch das Anbringen einer Verzahnung an der konkaven Innenseite der Aussenschale wird eine besonders zuverlässige Verdrehsicherung geschaffen. Die Verzahnung lässt sich leicht an der Aussenschale anbringen und kann den jeweils vorliegenden Erfordernissen angepasst werden.

Bevorzugt wird die Verzahnung durch ein spanabhebendes Verfahren wie beispielsweise Fräsen oder Stossen hergestellt. In bestimmten Fällen ist aber auch die Herstellung durch einen Umformungsprozess oder durch Erodieren denkbar.

Die Verzahnung kann sich durchgehend über den insbesondere kreisförmig umlaufenden Bereich erstrecken. Dadurch ist ein vergleichsweise hohes Drehmoment von der Aussenschale auf die Innenschale übertragbar. Allerdings ist auch eine vergleichsweise hohe Einpresskraft zum Einsetzen der Innenschale in die Aussenschale erforderlich. Daher kann sich die Verzahnung auch mit Unterbrüchen über den insbesondere kreisförmig umlaufenden Bereich erstrecken. Dadurch kann eine Reduktion der zum Einsetzen der Innenschale in die Aussenschale erforderlichen Einpresskraft erzielt werden, was allerdings auf Kosten des übertragbaren Drehmomentes geht. Durch ein gut abgestimmtes Verhältnis zwischen den Bereichen mit Verzahnung und den Unterbrüchen kann die Verdrehsicherung so ausgelegt werden, dass sich bei minimaler Einpresskraft ein akzeptables Drehmoment von der Aussenschale auf die Innenschale übertragen lässt.

Der Radius des Fusskreises der Verzahnung kann mindestens 50%, vorzugsweise mindestens 60%, bevorzugterweise mindestens 70% des Gesamtradius der Aussenschale betragen. Je mehr sich der Radius des Fusskreises dem Gesamtradius der Aussenschale annähert, desto grösser wird das übertragbare Drehmoment. Unter Fusskreis wird derjenige Kreis verstanden, auf dem die tiefsten Stellen der Verzahnung liegen.

Die Aussenschale kann zumindest ein Rastelement zum axialen Einrasten der Innenschale in der Aussenschale aufweisen. Im Zusammenhang mit derartigen Rastelementen wird im Fachjargon auch von so genannten Schnappverbindungen gesprochen. Eine derartige Schnappverbindung erlaubt es insbesondere bei Gelenkpfannenimplantaten mit Innenschalen aus Kunststoff, dass die Innenschale beim Einsetzen in die Aussenschale in diese einschnappt bzw. einrastet und dadurch festgehalten wird. Eine Kombination einer derartigen Schnappverbindung mit einer erfindungsgemässen Verdrehsicherung ermöglicht es, eine Innenschale derart in der Aussenschale zu verankern, dass sie sowohl gegen ein Verdrehen als auch gegen ein Hinausfallen gesichert ist.

Der insbesondere kreisförmig umlaufende Bereich mit der Verzahnung kann anschliessend an einem axialen Rastelement der Aussenschale angeordnet sein. Durch die Kombination von Rastelement und Verzahnung kann ein kompaktes Strukturelement auf der konkaven Innenseite der Aussenschale geschaffen werden, welches die Innenschale gleichzeitig gegen ein Verdrehen und ein Hinausfallen sichert.

Die Verzahnung kann ein Zahnprofil mit einer Höhe aufweisen, welche mindestens das 1,5 bis 2-fache der gewünschten Eindringtiefe hat. Durch dieses Verhältnis ist sichergestellt, dass das beim Einpressen verdrängte Material der Innenschale Platz findet und damit nicht zu einer Erhöhung der Einpresskraft führt.

Das Zahnprofil der Verzahnung kann dreieckig oder trapezförmig sein und einen Winkel α von 30° bis 120°, vorzugsweise von 45° bis 110°, bevorzugterweise von 60° bis 100°, einschliessen. Derartige Geometrien lassen sich gut an der konkaven Innenseite der Aussenschale anbringen. Sie weisen beim Eindringen der Verzahnung in die Aussenseite der Innenschale eine günstige Charakteristik auf. Zudem verhindert ein derartiges Zahnprofil in der Regel auf besonders zuverlässige Weise eine relative Rotationsbewegung zwischen dem Einsatz und der Aussenschale.

Die Flanken der Verzahnung können parallel zur Längsmittelachse der Aussenschale ausgerichtet sein. Diese Ausgestaltung ermöglicht es, dass der mit der Verzahnung in Kontakt kommende Bereich der Innenschale zylindrisch ausgebildet sein kann.

Die Verzahnung kann bezogen auf die Axialrichtung eine Breite von 10 mm bis 0.1 mm, vorzugsweise von 5 mm bis 0.5 mm, bevorzugterweise 1.6 mm bis 0.8 mm aufweisen.

Der umlaufende Bereich mit der Verzahnung kann in einem zylindrischen Abschnitt der konkaven Innenseite der Aussenschale angeordnet sein, der an einen konischen Abschnitt anschliesst. Durch das Anbringen eines konischen Abschnitts an der konkaven Innenseite der Aussenschale kann ein Verkippen der Innenschale während des Montierens im Operationssaal wirkungsvoll vermieden werden.

Derartige Aussenschalen sind je nach Ausführung sowohl als Erstersatz für ein Kugelgelenk als auch als Revisionsschalen geeignet.

Die vorliegende Erfindung betrifft ein Gelenkpfannenimplantat umfassend eine Aussenschale der oben beschriebenen Art sowie eine in die Aussenschale einsetzbare Innenschale. Die Härte der Aussenschale ist dabei grösser als diejenige der Innenschale. Dadurch ist bei Einsetzen der Innenschale in die Aussenschale eine Hemmung der relativen Rotationsfähigkeit der beiden Schalen durch Verdrängen des Materials der Innenschale durch die Verzahnung der Aussenschale erzielbar.

Bei einem derartigen Gelenkpfannenimplantat kann die Aussenschale aus Titan oder aus einer Titan-, Stahl-, Kobalt- oder Zirkon-Basislegierung gefertigt sein. Allerdings kann die Aussenschale auch aus einem Keramikmaterial gefertigt sein. Bei diesen Materialien handelt es sich um bei der Implantatherstellung gängige Werkstoffe. Sie zeichnen sich durch eine hervorragende Biokompatibilität aus und ermöglichen eine zuverlässige Verankerung der Aussenschale in einem Knochen eines Patienten.

Die Innenschale kann aus Polyethylen, insbesondere aus einem UHMWPE, oder aus einem UXPE, einem PEEK oder einem PEAK gefertigt sein. Unter UHMWPE wird im Zusammenhang mit der vorliegenden Anmeldung ein Polyethylen mit ultrahoher molekularer Masse verstanden. Bei den genannten Materialien handelt es sich um gängige Werkstoffe für die Herstellung von Laufflächen bei künstlichen Gelenken. Sie zeichnen sich durch einen besonders geringen Gleitwiderstand, eine lange Lebensdauer und eine gute Patientenverträglichkeit aus.

Allerdings ist es auch möglich, dass die Innenschale eine Verbundstruktur bestehend aus einem der oben genannten Kunststoffmaterialien und einem Metall oder einem Keramikmaterial ist. Bei einer derartigen Verbundstruktur ist die der Aussenschale zugewandte konvexe Aussenseite der Innenschale aus einem Kunststoffmaterial gefertigt, während die Lagerfläche des Gelenkes aus Metall oder einem Keramikmaterial ist. Derartige Verbundstrukturen lassen sich hervorragend in eine erfindungsgemässe Aussenschale einpressen und im Vergleich zu einer reinen Kunststoffinnenschale ein besseres Gleit- und Abriebverhalten der Lagerfläche auf.

Weitere Vorteile und Einzelmerkmale der Erfindung ergeben sich aus der nachfolgenden Beschreibung eines Ausführungsbeispiels und aus den Zeichnungen.

Es zeigen schematisch:
- Figur 1:: Perspektivische Darstellung einer Aussenschale für ein Gelenkpfannenimplantat;
- Figur 2:: Perspektivische Darstellung einer Innenschale zur Verwendung mit einer Aussenschale;
- Figur 3:: Draufsicht auf eine Aussenschale;
- Figur 4:: Vergrösserung des Teilbereichs B gemäss Figur 3;
- Figur 5:: Schematische Vergrösserung des Teilbereichs D gemäss Figur 4;
- Figur 6:: Schnittansicht einer Aussenschale durch die Schnittebene A gemäss Figur 3;
- Figur 7:: Vergrösserung des Teilbereichs C gemäss Figur 6;
- Figur 8:: Perspektivische Darstellung einer Innenschale eingesetzt in eine Aussenschale;
- Figur 9:: Schnittansicht einer Innenschale eingesetzt in eine Aussenschale;
- Figur 10:: Schnittansicht eines alternativen Ausführungsbeispiels einer Aussenschale;
- Figur 11:: Schnittansicht eines alternativen Ausführungsbeispiels einen Gelenkpfannenimplantats unter Verwendung der Aussenschale gemäss Figur 10;
- Figur 12:: Vergrösserung des Teilbereichs E gemäss Figur 10;
- Figur 13:: Vergrösserung des Teilbereichs F gemäss Figur 11;
- Figur 14:: Vergrösserung des Teilschnitts durch die Ebene G gemäss Figur 11 und;
- Figur 15:: Eine perspektivische und schematische Darstellung des Eingriffs von zwei Zähnen einer Verzahnung in das Material der Innenschale.

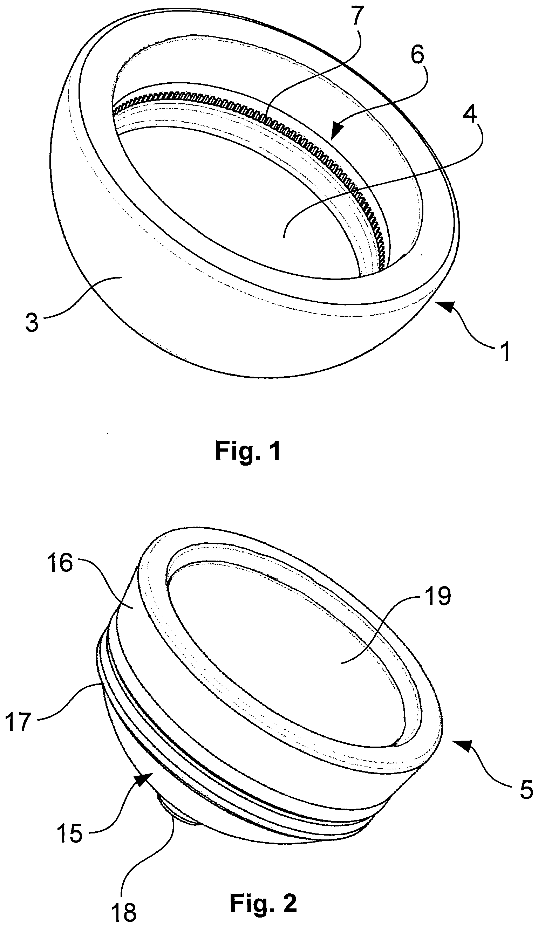

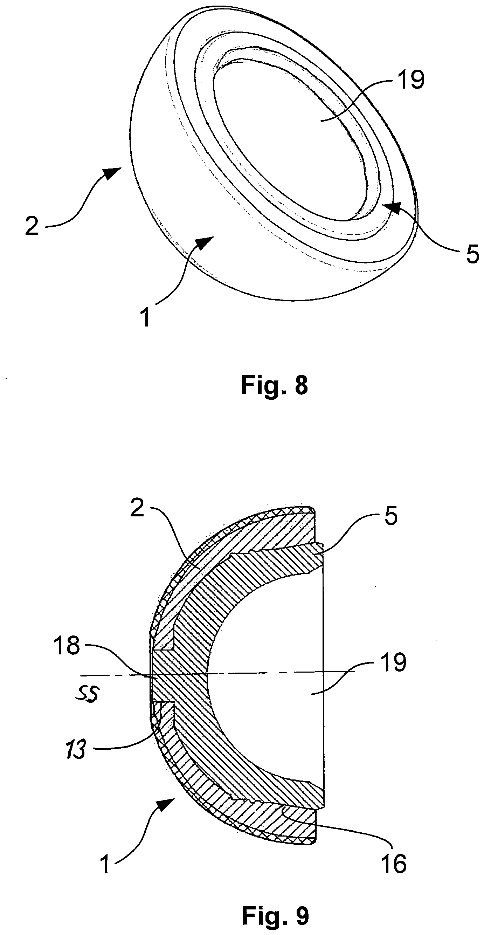

Wie aus Figur 1 hervorgeht, weist eine Aussenschale 1 für ein Gelenkpfannenimplantat 2 (Fig. 9) eine konvexe Aussenseite 3 und eine konkave Innenseite 4 auf. Die Innenseite 4 hat einen kreisförmig umlaufenden Bereich 6, welcher mit einer Verzahnung 7 versehen ist.

Wie Figur 2 zu entnehmen ist, weist eine Innenschale 5 für ein erfindungsgemässes Gelenkpfannenimplantat 2 auf ihrer konkaven Innenseite eine Gelenklagerfläche 19 auf. Die konvexe Aussenseite 15 ist in verschiedene Teilbereiche unterteilt. In Äquatornähe weist die gezeigte Innenschale 5 einen konischen Bereich 16 auf, an dessen polnahen Randbereich ein Rastelement 17 angeordnet ist. Am Pol der Innenschale 5 ist ein Fortsatz 18 angebracht. Sowohl der konische Bereich 16 als auch der Fortsatz 18 dienen dazu, ein Verkippen der Innenschale 5 in der Aussenschale 1 zu verhindern.

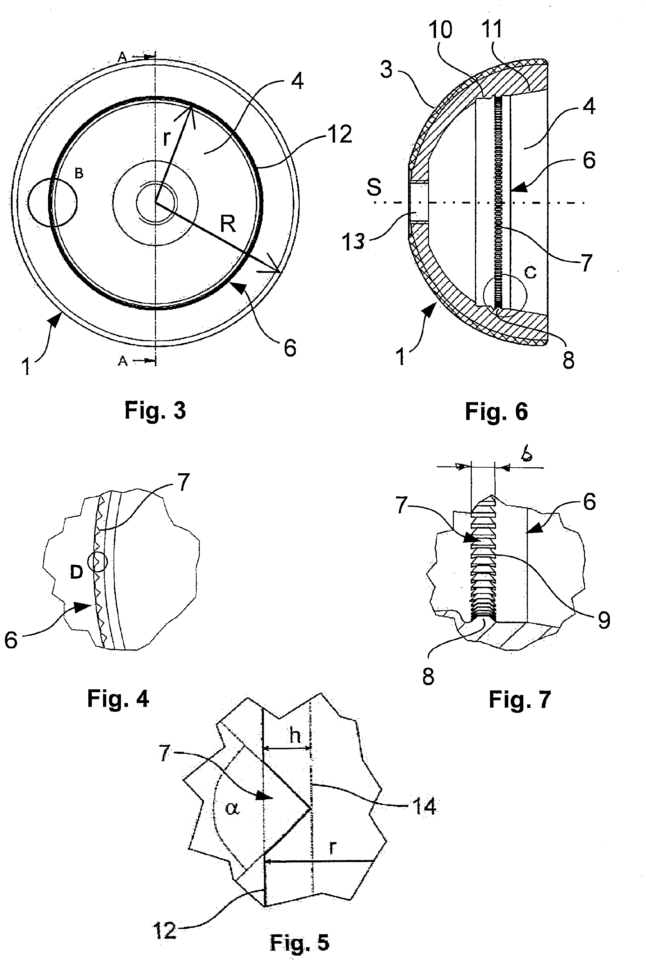

Aus Figur 3 wird deutlich, dass beim gezeigten Ausführungsbeispiel die Verzahnung 7 relativ weit aussen an der konkaven Innenseite 4 der Aussenschale 1 angebracht ist. Der Radius r des Fusskreises 12 der Verzahnung 7 beträgt hier ca. 70% des Gesamtradius R der Aussenschale 1.

Aus Figur 4 geht hervor, dass beim diskutierten Ausführungsbeispiel ein dreieckförmiges Zahnprofil für die Verzahnung verwendet wird, wobei die Dreiecke leicht voneinander beabstandet sind.

Bei Figur 5 handelt es sich um eine schematische Darstellung des Zahnprofils gemäss Figur 4. In das Zahnprofil sind der Fusskreis 12 und der Kopfkreis 14 gestrichelt eingezeichnet. Ebenfalls ist der durch das Zahnprofil eingeschlossene Winkel α ausgewiesen. Ferner ist zu erkennen, dass die Höhe h des Zahnprofils definiert ist als die Differenz zwischen dem Radius des Kopfkreises 14 und desjenigen des Fusskreises 12.

Durch die Schnittansicht gemäss Figur 6 werden weitere Einzelheiten der erfindungsgemässen Aussenschale 1 ersichtlich. Es zeigt sich, dass die konkave Innenseite 4 der Aussenschale 1 in verschiedene Bereiche unterteilt ist. So ist der Bereich 10, in welchem auch das mit der Verzahnung 7 ausgestattete axialen Rastelement 8 angeordnet ist, stufenweise ausgestaltet. Dagegen ist der Bereich 11 konisch geformt. Die Aussenschale 1 weist an ihrem Pol eine Öffnung 13 auf. Die Längsmittelachse der Aussenschale ist mit S bezeichnet.

Aus Figur 7 geht die Ausgestaltung des mit einer Verzahnung 7 versehenen Rastelementes 8 hervor. Es ist zu erkennen, dass die Verzahnung 7 die Breite b hat, welche im vorliegenden Ausführungsbeispiel mit derjenigen des Rastelementes identisch ist. Ferner ist zu erkennen, dass die Verzahnung 7 hier ein trapezförmiges Querprofil aufweist.

In Figur 8 ist ein erfindungsgemässes Gelenkpfannenimplantat 2 mit Aussenschale 1 und Innenschale 5 perspektivisch dargestellt. Auf der konkaven Innenseite ist insbesondere die Gelenklagerfläche 19 zu sehen. Figur 9 zeigt einen Längsschnitt durch das besagte Gelenkpfannenimplantat 2. Es ist zu erkennen, dass der Fortsatz 18 am Pol der Innenschale 5 in die dafür vorgesehene Öffnung 13 in der Aussenschale 1 eingreift. Die gemeinsame Hauptachse (Längsmittelachse) von Aussenschale und Innenschale ist mit SS bezeichnet.

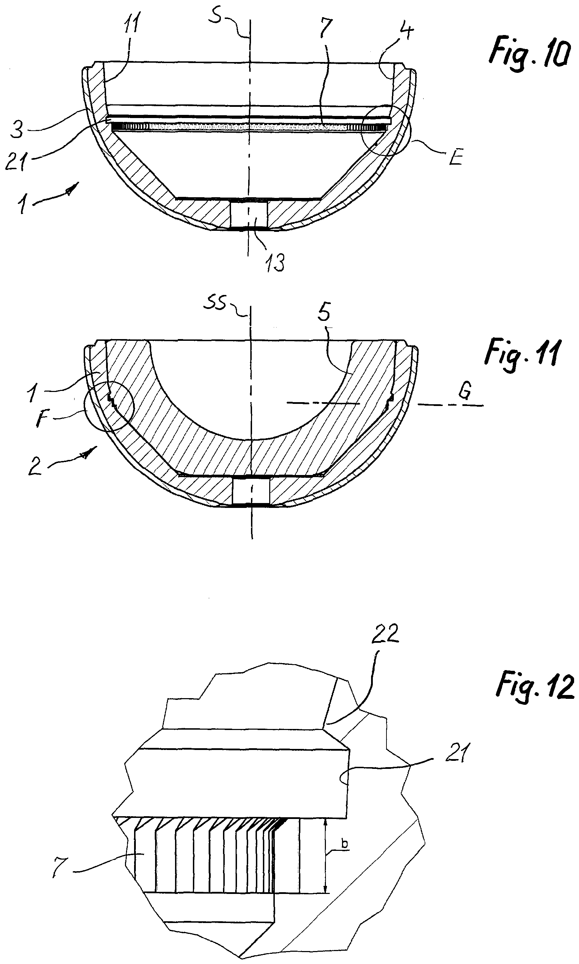

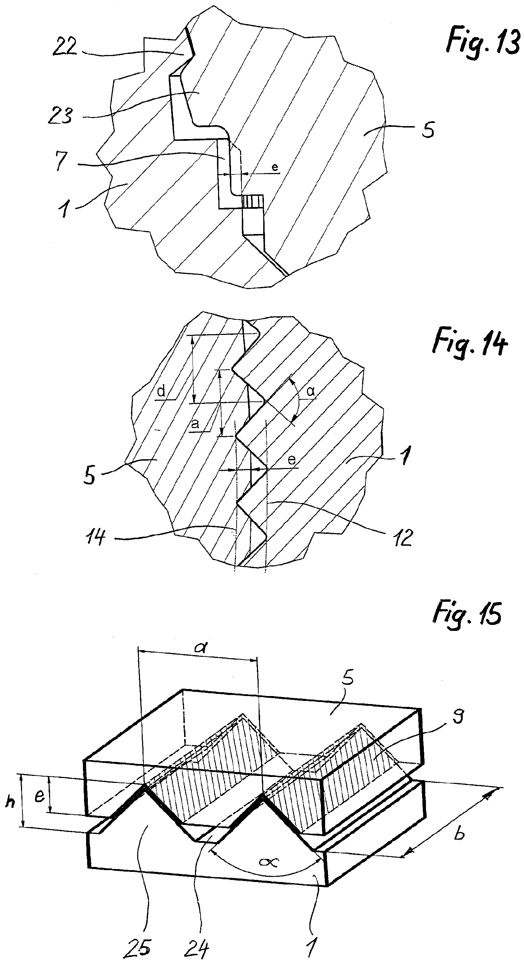

Die Figuren 10 und 11 zeigen ein abgewandeltes Ausführungsbeispiel eines Gelenkpfannenimplantats, bei dem die Aussenschale eine andere Innenkontur aufweist. Im Gegensatz zur Aussenschale gemäss Figur 6 ist die Verzahnung 7 in einem anderen Bereich angeordnet. Auf den konischen Einlaufbereich 11 an der Innenseite 4 folgt eine ebenfalls konische Hinterschneidung 21, deren Konfiguration in Figur 12 genauer ersichtlich ist. Die Schulter 22 zwischen der Hinterschneidung 21 und dem konischen Bereich 11 bildet das Rastelement der Aussenschale, hinter welchem eine korrespondierende Materialschulter 23 der Innenschale einrastet (Figur 13). Unmittelbar unter der Hinterschneidung 21 schliesst sich ein umlaufender zylindrischer Bereich an, an welchem die Verzahnung 7 angebracht ist. Die Verzahnung erstreckt sich dabei über die Breite b und damit nicht über den gesamten zylindrischen Bereich. Im Gegensatz zur Innenschale gemäss Figur 9 weist die Innenschale gemäss Figur 11 keinen Materialfortsatz auf, welcher in die Öffnung 13 eingreift. Aus Figur 13 ist die Situation im Bereich der Rastverbindung und der Verzahnung genauer ersichtlich. Demnach ist ein zylindrischer Bereich an der Innenschale gegenüber dem Kopfkreis der Verzahnung mit einem Übermass versehen, so dass sich je nach Materialtoleranzen eine Eindringtiefe e der einzelnen Zähne in das Material der Innenschale 5 ergibt. Diese Eindringtiefe ist in Figur 14 nachmals dargestellt, wobei die Zahnflanken zwischen sich einen Winkel α von 90° einschliessen. Der Flankenwinkel bestimmt die Zahnstärke d im Bereich des Fusskreises 12 und der Abstand zwischen zwei Zähnen im Bereich des Kopfkreises 14 ist mit dem Mass a angegeben. Wie aus Figur 1 deutlich ersichtlich ist, verbleibt nach dem Einpressen der Innenschale 5 zwischen zwei Zahnflanken ein Hohlraum 24 der ausreichend gross bemessen sein muss, dass das durch die Zähne verdrängte Material der Innenschale ausweichen kann.

In Figur 15 sind die wesentlichen Parameter der Verzahnung nochmals schematisch dargestellt. Die Eindringtiefe e sollte 0,1 mm bis 1 mm nicht überschreiten. Angestrebt wird eine möglichst grosse Anzahl Zähne 25 mit gleichmässigem Abstand a zueinander. Diese Zähne haben eine Höhe h, wobei zwischen e und h eine Differenz verbleibt, so dass zwischen benachbarten Zahnflanken 9 ein Hohlraum 24 verbleibt. b ist die Breite der Verzahnung oder genauer gesagt die Breite der Eindringung der einzelnen Zähne in das Material der Innenschale 5. Wie einleitend dargestellt, nimmt das übertragbare Drehmoment zu, je grösser bei gegebener Eindringtiefe e die Breite b der Verzahnung ist. Dagegen wird die Einpresskraft im Wesentlichen durch die Eindringtiefe e bestimmt. Anhand eines durch Medizinalnormen vorgegebenen Drehmoments von z.B. grösser als 12 Nm, das die Innenschale auf die Aussenschale übertragen können muss, werden in Abhängigkeit von unterschiedlichen Schalengrössen die notwendigen Dimensionen der Verzahnung bestimmt.

## Patentansprüche

1. Gelenkpfannenimplantat (2), insbesondere Hüftgelenkpfannenimplantat, umfassend eine Aussenschale (1) mit einer konvexen Aussenseite (3) und einer konkaven Innenseite (4), welche in einem kreisförmig umlaufenden Bereich (6) eine Verzahnung (7) aufweist, wobei die Flanken (9) der Verzahnung parallel zu einer Längsmittelachse (S) der Aussenschale (1) ausgerichtet sind und wobei das Zahnprofil eine Zahnhöhe (h) aufweist, welche definiert ist als Differenz zwischen dem Radius des Kopfkreises und dem Radius des Fusskreises der Verzahnung, und eine in die Aussenschale eingesetzte Innenschale (5), wobei die Härte der Aussenschale grösser ist als diejenige der Innenschale und wobei der mit der Verzahnung in Kontakt kommende Bereich der Innenschale relativ zum Kopfkreis der Verzahnung ein Übermass hat, sodass eine Hemmung der relativen Rotationsfähigkeit der beiden Schalen durch Verdrängen des Materials der Innenschale durch die Verzahnung erzielbar ist, **dadurch gekennzeichnet, dass** sich die Verzahnung (7) durchgehend, mit gleichmässiger Teilung über den kreisförmig umlaufenden Bereich (6) erstreckt, so dass die Verzahnung (7) einer sich über 360° erstreckenden Innenverzahnung entspricht, wobei die Verzahnung (7) mit einer Zahneindringtiefe (e) in das Material der Innenschale (5) eindringt, welche zwischen 0.1 bis 1.0 mm liegt, wobei zwischen den Seitenflanken benachbarter Zähne und der in die Verzahnung (7) eingesetzten Innenschale (5) ein Hohlraum (24) verbleibt, und die Gesamtanzahl Zähne der Verzahnung (7) und die Breite (b) der Zähne bezogen auf die Längsmittelachse (S) derart gewählt werden, dass die Hemmung der Rotationsfähigkeit ein vorgegebenes Solldrehmoment von 10 Nm übertrifft.

2. Gelenkpfannenimplantat nach Anspruch 1, wobei die Aussenschale (1) aus Titan oder aus einer Titan-, Stahl-, Kobalt-
, oder Zirkon-Basislegierung gefertigt ist.

3. Gelenkpfannenimplantat nach Anspruch 1 oder 2, wobei die Innenschale (5) aus einem Polyethylen, insbesondere aus einem UHMWPE oder aus einem UXPE, oder aus einem PEEK oder aus einem PEAK gefertigt ist.

4. Gelenkpfannenimplantat nach einem der Ansprüche 1 bis 3, wobei die Aussenschale (1) und die Innenschale (5) korrespondierende axiale Rastelemente (8, 17) aufweisen, an denen sie bezogen auf die gemeinsame Längsmittelachse (S) miteinander verrastet sind.

5. Gelenkpfannenimplantat nach Anspruch 4, wobei der umlaufende Bereich mit der Verzahnung in einem zylindrischen Abschnitt der konkaven Innenseite (4) der Aussenschale (1) angeordnet ist, der sich bezogen auf die gemeinsame Längsmittelachse (S) an die korrespondierenden Rastelemente (8, 17) anschliesst.

6. Gelenkpfannenimplantat nach einem der Ansprüche 1 bis 5, wobei die Zahneindringtiefe (e) zwischen 0.15 mm bis 0.3 mm liegt.

7. Gelenkpfannenimplantat nach einem der Ansprüche 1 bis 6, wobei die einzelnen Zähne der Verzahnung einen Winkel von vorzugsweise 45° bis 100° einschliessen.

8. Gelenkpfannenimplantat nach Anspruch 7, wobei die Seitenflanken (9) der Zähne ebene Flächen sind.

9. Gelenkpfannenimplantat nach einem der Ansprüche 1 bis 8, wobei der umlaufende Bereich mit der Verzahnung (7) bezogen auf die Längsmittelachse (S) näher am Äquator der Aussenschale (1) liegt als an deren Scheitelpunkt.

10. Gelenkpfannenimplantat nach einem der Ansprüche 1 bis 9, wobei die Innenschale (5) aus einem Kunststoffmaterial, insbesondere aus einem Polyethylen, mit einer Shore-Härte D zwischen 50 und 85 besteht.

11. Gelenkpfannenimplantat nach einem der Ansprüche 1 bis 10, wobei mittels der Verzahnung ein Drehmoment von mehr als 12 Nm übertragbar ist.

## Claims

1. Joint socket implant (2), in particular an acetabular implant, comprising an outer shell (1) with a convex outer face (3) and a concave inner face (4), which has a toothing system (7) in a circular circumferential region (6), wherein the flanks (9) of the toothing system are oriented parallel to a longitudinal central axis (S) of the outer shell (1), and wherein the tooth profile has a tooth height (h) defined as the difference between the radius of the tip circle and the radius of the root circle of the toothing system, and an inner shell (5) inserted into the outer shell, wherein the hardness of the outer shell is greater than that of the inner shell, and wherein the region of the inner shell coming into contact with the toothing system has an oversize relative to the tip circle of the toothing system, such that an inhibition of the rotatability of the two shells relative to each other can be achieved by the material of the inner shell being displaced by the toothing system, **characterized in that** the toothing system (7) extends continuously, with uniform pitch, about the circular circumferential region (6), such that the toothing system (7) corresponds to an inner toothing extending through 360°, wherein the toothing system (7) penetrates into the material of the inner shell (5) with a tooth penetration depth (e) of between 0.1 and 1.0 mm, wherein a hollow space (24) remains between the side flanks of adjacent teeth and the inner shell (5) inserted into the toothing system (7), and the total number of teeth of the toothing system (7) and the width (b) of the teeth relative to the longitudinal central axis (S) are chosen in such a way that the inhibition of the rotatability exceeds a predefined setpoint torque of 10 Nm.

2. Joint socket implant according to Claim 1, wherein the outer shell (1) is produced from titanium or from a titanium-based, steel-based, cobalt-based or zirconium-based alloy.

3. Joint socket implant according to Claim 1 or 2, wherein the inner shell (5) is produced from a polyethylene, in particular from a UHMWPE or from a UXPE, or from a PEEK or from a PEAK.

4. Joint socket implant according to one of Claims 1 to 3, wherein the outer shell (1) and the inner shell (5) have corresponding axial locking elements (8, 17), at which they are locked onto each other relative to the common longitudinal central axis (S).

5. Joint socket implant according to Claim 4, wherein the circumferential region with the toothing system is arranged in a cylindrical portion of the concave inner face (4) of the outer shell (1) adjacent to the corresponding locking elements (8, 17), relative to the common longitudinal central axis (S) .

6. Joint socket implant according to one of Claims 1 to 5, wherein the tooth penetration depth (e) is between 0.15 mm and 0.3 mm.

7. Joint socket implant according to one of Claims 1 to 6, wherein the individual teeth of the toothing system enclose an angle of preferably 45° to 100°.

8. Joint socket implant according to Claim 7, wherein the side flanks (9) of the teeth are plane surfaces.

9. Joint socket implant according to one of Claims 1 to 8, wherein, relative to the central longitudinal axis (S), the circumferential region with the toothing system (7) lies closer to the equator of the outer shell (1) than to the pole.

10. Joint socket implant according to one of Claims 1 to 9, wherein the inner shell (5) is made of a plastic material, in particular of a polyethylene, with a Shore hardness D of between 50 and 85.

11. Joint socket implant according to one of Claims 1 to 10, wherein a torque of more than 12 Nm can be transmitted by means of the toothing system.

## Revendications

1. Implant cotyloïdien (2), en particulier implant cotyloïdien de la hanche, comportant une coque extérieure (1) dotée d'un côté extérieur convexe (3) et d'un côté intérieur concave (4), lequel comprend une denture (7) dans une région périphérique circulaire (6), dans lequel les flancs (9) de la denture sont orientés parallèlement à un axe médian longitudinal (S) de la coque extérieure (1) et dans lequel le profil de dent présente une hauteur de dent (h), laquelle est définie comme la différence entre le rayon du cercle de tête et le rayon du cercle de pied de la denture, et une coque intérieure (5) insérée dans la coque extérieure, dans lequel la dureté de la coque extérieure est supérieure à celle de la coque intérieure et dans lequel la région de la coque intérieure venant en contact avec la denture présente une surmesure par rapport au cercle de tête de la denture, de sorte qu'un blocage de l'aptitude à la rotation relative des deux coques peut être obtenu par déplacement de la matière de la coque intérieure par la denture, **caractérisé en ce que** la denture (7) s'étend de manière continue avec un pas uniforme sur la région périphérique circulaire (6), de sorte que la denture (7) correspond à une denture intérieure s'étendant sur 360°, la denture (7) pénétrant dans la matière de la coque intérieure (5) sur une profondeur de pénétration de dent (e), laquelle est comprise entre 0,1 et 1,0 mm, une cavité (24) demeurant entre les flancs latéraux de dents adjacentes et la coque intérieure (5) insérée dans la denture (7), et le nombre total de dents de la denture (7) et la largeur (b) des dents par rapport à l'axe médian longitudinal (S) étant sélectionnés de telle sorte que le blocage de l'aptitude à la rotation dépasse un couple de consigne prédéfini de 10 Nm.

2. Implant cotyloïdien selon la revendication 1, dans lequel la coque extérieure (1) est fabriquée à partir de titane ou à partir d'un alliage à base de titane, d'acier, de cobalt ou de zircon.

3. Implant cotyloïdien selon la revendication 1 ou 2, dans lequel la coque intérieure (5) est fabriquée à partir d'un polyéthylène, en particulier à partir d'un UHMWPE ou à partir d'un UXPE, ou à partir d'un PEEK ou à partir d'un PEAK.

4. Implant cotyloïdien selon l'une des revendications 1 à 3, dans lequel la coque extérieure (1) et la coque intérieure (5) comprennent des éléments d'encliquetage axiaux (8, 17) correspondants, au niveau desquels elles sont encliquetées l'une avec l'autre par rapport à l'axe médian longitudinal commun (S).

5. Implant cotyloïdien selon la revendication 4, dans lequel la région périphérique dotée de la denture est disposée dans une partie cylindrique du côté intérieur concave (4) de la coque extérieure (1), qui se raccorde aux éléments d'encliquetage (8, 17) correspondants par rapport à l'axe médian longitudinal commun (S).

6. Implant cotyloïdien selon l'une des revendications 1 à 5, dans lequel la profondeur de pénétration de dent (e) est comprise entre 0,15 mm et 0,3 mm.

7. Implant cotyloïdien selon l'une des revendications 1 à 6, dans lequel les dents individuelles de la denture forment un angle de préférence de 45° à 100°.

8. Implant cotyloïdien selon la revendication 7, dans lequel les flancs latéraux (9) des dents sont des surfaces planes.

9. Implant cotyloïdien selon l'une des revendications 1 à 8, dans lequel la région périphérique dotée de la denture (7) est située, par rapport à l'axe médian longitudinal (S), plus près de l'équateur de la coque extérieure (1) que de son sommet.

10. Implant cotyloïdien selon l'une des revendications 1 à 9, dans lequel la coque intérieure (5) est constituée d'une matière synthétique, en particulier d'un polyéthylène, présentant une dureté Shore D comprise entre 50 et 85.

11. Implant cotyloïdien selon l'une des revendications 1 à 10, dans lequel un couple supérieur à 12 Nm peut être transmis au moyen de la denture.
